(19) Europäisches Patentamt
European Patent Office
Office européen des brevets

(11) **EP 3 629 065 A1**

(12) **EUROPÄISCHE PATENTANMELDUNG**

(43) Veröffentlichungstag:
**01.04.2020 Patentblatt 2020/14**

(51) Int Cl.:
*G01T 1/29* (2006.01)    *A61B 6/03* (2006.01)

(21) Anmeldenummer: **18197600.2**

(22) Anmeldetag: **28.09.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Siemens Healthcare GmbH**
**91052 Erlangen (DE)**

(72) Erfinder:
• **Ergler, Thorsten**
**91054 Erlangen (DE)**
• **Kreisler, Björn**
**91353 Hausen (DE)**

(54) **VERFAHREN ZUR KORREKTUR VON DETEKTORELEMENTMESSWERTEN, RÖNTGENDETEKTOR UND MEDIZINISCHES GERÄT**

(57)    Die Erfindung betrifft ein Verfahren (10) zur Korrektur von einem ersten Detektorelementmesswert und von einem zweiten Detektorelementmesswert eines Detektorelements eines Röntgendetektors aufweisend die Schritte:
- Aufnahme (11) des ersten Detektorelementmesswerts bei einer ersten Fokusposition und des zweiten Detektorelementmesswerts einer von der ersten Fokusposition verschiedenen zweiten Fokusposition, und
- Korrektur (13) des ersten Detektorelementmesswerts und des zweiten Detektorelementmesswerts, wobei eine durch den Wechsel von der ersten Fokusposition zur zweiten Fokusposition bedingte Schwankung zwischen dem ersten Detektorelementmesswert und dem zweiten Detektorelementmesswert im Wesentlichen ausgeglichen wird.

FIG 1

EP 3 629 065 A1

**Beschreibung**

Die Erfindung betrifft ein Verfahren zur Korrektur von Detektorelementmesswerten sowie Röntgendetektor und medizinisches Gerät dazu.

In der, insbesondere medizinischen, Röntgenbildgebung, beispielsweise in der Computertomographie, der Angiographie oder der Radiographie, können zählende direkt-konvertierende Röntgendetektoren oder integrierende indirekt-konvertierende Röntgendetektoren verwendet werden.

Die Röntgenstrahlung oder die Photonen können in direkt-konvertierenden Röntgendetektoren durch ein geeignetes Konvertermaterial in elektrische Pulse umgewandelt werden. Als Konvertermaterial können beispielsweise CdTe, CZT, CdZnTeSe, CdTeSe, CdMnTe, InP, TIBr2, HgI2, GaAs oder andere verwendet werden. Die elektrischen Pulse werden von einer Auswerteelektronik, beispielsweise einem integrierten Schaltkreis (Application Specific Integrated Circuit, ASIC), bewertet. In zählenden Röntgendetektoren wird einfallende Röntgenstrahlung durch Zählen der elektrischen Pulse, welche durch die Absorption von Röntgenphotonen im Konvertermaterial ausgelöst werden, gemessen. Die Höhe des elektrischen Pulses ist in der Regel proportional zur Energie des absorbierten Röntgenphotons. Dadurch kann eine spektrale Information durch den Vergleich der Höhe des elektrischen Pulses mit einem Schwellwert extrahiert werden.

Aus der Druckschrift DE 10 2014 215 548 A1 ist ein Verfahren zur Korrektur einer Verteilung von Intensitätswerten, ein Verfahren zur Rekonstruktion eines Röntgenbildes sowie ein Tomographiegerät bekannt. Dabei wird von einer durch einen Röntgendetektor mit einem Kollimator aufgenommenen Verteilung von Intensitätswerten ausgegangen, wobei die Intensitätswerte jeweils einem Detektorelement des Röntgendetektors zugeordnet sind. Die Instabilität des Fokus einer Röntgenquelle lässt sich korrigieren, indem eine erste sowie eine zweite Auswahl von Intensitätswerten miteinander verglichen werden, wobei die erste Auswahl sowie die zweite Auswahl der Intensitätswerte auf unterschiedlichen Seiten einer Absorberwand des Kollimators gelegenen Detektorelementen zugeordnet sind. Denn durch einen solchen Vergleich kann eine Asymmetrie der Abschattung durch die Absorberwand ermittelt werden, wobei die Asymmetrie ein Maß für die Instabilität des Fokus der Röntgenquelle ist. Die Korrektur erfolgt dann für wenigstens einen Teil der Intensitätswerte basierend auf dem Vergleich. Durch die Korrektur wird die asymmetrische Abschattung kompensiert.

Aus der Druckschrift DE 10 2014 225 399 A1 ist ein Verfahren zum Korrigieren eines erfassten Makropixelsignals eines Röntgendetektors mit einer Mehrzahl von Pixeln bekannt. Die Mehrzahl von Pixeln ist jeweils zu mindestens einem Makropixel zusammengefasst und kann jeweils Einzelsignale erfassen. Bei dem Verfahren wird ein gewichtetes Makropixelsignal mit verbesserter Signalstabilität aber reduzierter Dosiseffizienz ermittelt. Auf Basis des erfassten Makropixelsignals und des gewichteten Makropixelsignals wird eine die relative Signaldrift des ungewichteten Makropixelsignals im Vergleich zu dem gewichteten Makropixelsignal angebende Größe ermittelt. Zusätzlich wird eine zeitlich gefilterte relative Signaldrift auf Basis der relativen Signaldrift ermittelt. Schließlich wird ein um die zeitlich gefilterte relative Signaldrift korrigiertes Makropixelsignal ermittelt.

Aus der Druckschrift DE 10 2014 222 855 A1 ist ein Verfahren zum Einstellen der Erfassung eines Makropixelsignals eines Röntgendetektors mit einer Mehrzahl von Pixeln bekannt. Die Mehrzahl von Pixeln ist jeweils zu mindestens einem Makropixel zusammengefasst und kann jeweils Einzelsignale erfassen. Bei dem Verfahren werden zunächst die geometrische Effizienz und der Signaldriftfaktor der einzelnen Pixel ermittelt. Weiterhin wird ein Zieldriftwert ermittelt. Ferner wird auch ein Parameter, welcher einen Kompromiss aus einer erlaubten Drift der Makropixelsignale und der erzielbaren Dosiseffizienz einstellt, festgelegt. Auf Basis der ermittelten Parameter wird die Gewichtung der einzelnen Pixelsignale unter Berücksichtigung einer sowohl die Signaldrift als auch die Dosisnutzung des resultierenden Makropixelsignals berücksichtigenden Funktion in Abhängigkeit von den Gewichtungen der Pixelsignale ermittelt. Schließlich wird eine gewichtete Addition der einzelnen Pixelsignale zu Makropixelsignalen auf Basis der ermittelten Gewichtungen festgelegt.

Herkömmliche, indirekt-konvertierende Röntgendetektoren für Computertomographiesysteme weisen in der Regel ein Streustrahlraster (Abkürzung: ASG) auf, welches den gleichen Pitch aufweist wie die Pixelierung des Konvertermaterials. Somit erzeugt eine Verschiebung der Ausrichtung vom Fokus bzw. der Fokusposition der Röntgenquelle gegenüber der Pixelierung des Röntgendetektors bzw. dem Konvertermaterial bei exaktem Aufbau in jedem Pixel bzw. jedem Detektorelement eine gleichmäßige Verschiebung: ein Pixel verliert auf der einen Pixelseite ähnlich viel Primär-Strahlung der Röntgenquelle durch Abschattung, wie auf der anderen Seite hinzugewonnen wird. Bei direkt-konvertierenden, zählenden Detektoren sind die einzelnen (Makro-)Pixel in mehrere Subpixel bzw. Detektorelemente unterteilt, um die auftretenden hohen Röntgenflüssen verarbeiten zu können. Somit sind einem Schacht bzw. einer Gitteröffnung des Streustrahlengitters mehrere Detektorelemente zugeordnet. Da nicht jedes Detektorelement auf jeder Seite eine Wand des Streustrahlengitters aufweist, kann es bei einer Verschiebung der Ausrichtung zwischen Röntgenröhre/ Fokus und Detektorpixelierung bzw. einer Verschiebung der Fokusposition dazu kommen, dass einzelne Detektorelemente ungleichmäßig von der Verschiebung betroffen sind. Explizit trifft die Abschattung in der Regel nur jene Detektorelemente, die direkt an der entsprechenden Wand des Streustrahlengitters angeordnet sind. Dieser Effekt kann bei Messungen mit einem Springfokus für jedes Reading bzw. jede Messung anders aussehen und kann somit zu sehr unterschiedlichen Messwerten bei unterschiedlichen Fokuspositionen führen. Insbesondere tritt dieser Effekt bei Rotation des Röntgenquellen-Röntgendetektor-Paares, insbesondere im Rotor eines Computertomographiesystems, auf. Die Fliehkräfte zeigen immer radial nach außen, während die Gewichtskraft der einzelnen Komponenten, beispielsweise Röntgenquelle oder Röntgendetektor, immer nach unten zeigt. Es wirkt also auf die Komponenten während des Umlaufs eine Kraft, die mit 1g während eines Umlaufs variiert. Dies kann dazu führen, dass der Röntgendetektor bzw. die Detektorvorrichtung mit mehreren Röntgendetektoren und weiteren Ausleseeinheiten dazu sich etwas verformt und so die Ausrichtung zwischen Fokuspunkt und Detektorelement schwankt. In Kombination mit den Wänden des Streustrahlengitters können die Folgen dynamische Abschattungseffekte, die zu einem instabilen Signal bzw. variablen Messungen führen, und Artefakte bzw. eine verminderte Bildqualität verursachen.

Es ist Aufgabe der Erfindung, ein Verfahren zur Korrektur von mindestens zwei Detektorelementmesswerten, einen Röntgendetektor, ein medizinisches Gerät und eine Verwendung des Röntgendetektors anzugeben, welche einen Ausgleich von Schwankungen in den Detektorelementmesswerten durch den Wechsel der Fokusposition ermöglichen.

Die Aufgabe wird erfindungsgemäß gelöst durch ein Verfahren nach Anspruch 1, einen Röntgendetektor nach Anspruch 10, ein medizinisches Gerät nach Anspruch 11 und eine Verwendung des Röntgendetektors nach Anspruch 13.

Die Erfindung betrifft ein Verfahren zur Korrektur von einem ersten Detektorelementmesswert oder / und von einem zweiten Detektorelementmesswert eines Detektorelements eines Röntgendetektors aufweisend die Schritte der Aufnahme und der Korrektur. Im Schritt der Aufnahme werden der erste Detektorelementmesswert bei einer ersten Fokusposition, insbesondere einer Röntgenquelle, und der zweite Detektorelementmesswert bei einer von der ersten Fokusposition verschiedenen zweiten Fokusposition, insbesondere der Röntgenquelle, aufgenommen. Im, bevorzugt nachfolgenden, Schritt der Korrektur werden der erste Detektorelementmesswert oder / und der zweite Detektorelementmesswert korrigiert, wobei eine durch den Wechsel von der ersten Fokusposition zur zweiten Fokusposition bedingte Schwankung zwischen dem ersten Detektorelementmesswert und den zweiten Detektorelementmesswert im Wesentlichen ausgeglichen werden. Bevorzugt werden der erste Detektorelementmesswert und der zweite Detektorelementmesswert korrigiert. Das erfindungsgemäße Verfahren kann bevorzugt auf die Korrektur von einer Mehrzahl von ersten oder / und zweiten Detektorelementmesswerten erweitert werden.

Der Röntgendetektor kann insbesondere als direkt-konvertierender Röntgendetektor ausgebildet sein. Alternativ bzw. zusätzlich kann der Röntgendetektor als indirektkonvertierender Röntgendetektor ausgebildet sein. Der erste und der zweite Detektorelementmesswert des insbesondere direkt-konvertierenden Röntgendetektors können jeweils Zählwerte oder Integralwerte basierend auf den detektierten Röntgenphotonen sein. Im Fall eines indirekt-konvertierenden Röntgendetektors können der erste und der zweite Detektorelementmesswert ein Intensitätswert basierend auf den detektierten Röntgenphotonen sein.

Der Röntgendetektor kann insbesondere ein Streustrahlengitter mit beispielsweise gitterförmig angeordneten Streustrahlenelementen aufweisen, wobei zwischen den Streustrahlenelementen Durchlassöffnungen angeordnet sind. Die Streustrahlenelemente können zu einem gemeinsamen entfernten Punkt, beispielsweise der ersten oder zweiten Fokusposition, ausgerichtet sein. Die Röntgenphotonen können durch die Durchlassöffnung auf das Detektorelement einfallen und als erster bzw. zweiter Detektorelementmesswert registriert werden. Es können mehrere Detektorelemente derart angeordnet sein, dass sie einer Durchlassöffnung gemeinsam zugeordnet sind. Der Röntgendetektor kann dabei bevorzugt ein direkt-konvertierender Röntgendetektor sein.

Die Röntgenquelle kann bevorzugt als Röntgenröhre ausgebildet sein. Die Röntgenquelle umfasst mehrere Fokuspositionen, zwischen denen gewechselt werden kann, beispielsweise mittels magnetischer Ablenkung oder mittels Sperrgitter. Dies bezeichnet man auch als Springfokus. Die Fokusposition kann beispielsweise der Position eines elektronischen oder optischen Fokus bzw. Brennfleck entsprechen. Der elektronische Brennfleck kann sich insbesondere an der Oberfläche der Anode der Röntgenquelle befinden. Der optische Brennfleck befindet sich in der Bildebene bzw. auf einer Detektionsfläche des Röntgendetektors. Die erste Fokusposition ist von der zweiten Fokusposition verschieden.

Der Wechsel von der ersten Fokusposition zur zweiten Fokusposition kann alternativ oder zusätzlich durch eine zeitlich veränderliche Schwankung entsprechend einer Funktion des Umlaufes oder / und der Rotationsgeschwindigkeit ausgebildet sein. Es können Schwankungen der Abschattung durch Verbiegen des Rotors bzw. der Gantry oder des Streustrahlengitters auftreten. Dabei ändert sich beispielsweise der Abstand oder / und auch die Lage des Fokuspunkts innerhalb der phi-z-Ebene bzw. die Anordnung des Streustrahlenelements des Streustrahlengitters zum Detektorelement. Dadurch kann eine Abschattung eines Detektorelements durch einen veränderten Einfall der Röntgenstrahlung auf das Streustrahlenelement bedingt sein. Dem Streustrahlengitter kann ein Fokus zugeordnet sein, insbesondere der Punkt auf den die Streustrahlenelemente ausgerichtet sind. Damit kann beispielsweise bei einer Verbiegung des Streustrahlengitters die erste Fokusposition des Streustrahlengitters zur zweiten Fokusposition des Streustrahlengitters verändert werden. Im Allgemeinen kann der Wechsel von der ersten Fokusposition zur zweiten Fokusposition eine Veränderung der, insbesondere optimalen, Ausrichtung von der Röntgenquelle und dem Röntgendetektor, oder von der Röntgenquelle und dem Streustrahlengitter, oder vom Streustrahlengitter und dem Röntgendetektor, oder von der Röntgenquelle, dem Streustrahlengitter und dem Röntgendetektor bezeichnen. Es kann der Röntgenquelle, dem Streustrahlengitter oder / und dem Röntgendetektor eine erste Fokusposition und eine zweite Fokusposition zugeordnet werden.

Die Fokusposition kann ferner durch eine Fokusgröße charakterisiert sein. Die Fokusposition kann eine Fokusgröße sein. Die Fokusposition kann eine Fokusgröße umfassen. Die Fokusgröße kann beispielsweise an die thermischen Randbedingungen der Röntgenquelle angepasst werden. Die Fokusgröße kann beispielsweise an die gewünschte Schärfe des Fokus angepasst werden.

Eine gewichtete Addition der Pixelantworten zu größeren bildgebungsrelevanten Pixelflächen kann zur Verbesserung der Detektorantwort des Röntgendetektors bzw. zur Reduktion der Drift- und Chargesharing-Einflüsse verwendet werden. Um diese Effekte auszugleichen können statische bzw. unveränderliche Additionsgewichte zur Korrektur verwendet werden. Der korrigierte Detektorelementmesswert $S_{ij}$ kann mit dem ersten Wichtungsfaktor $W_{ij}$ und dem eingehenden Detektorelementmesswert $P_{ij}$ des Detektorelements mit dem Index i für die Zeile und j für die Spalte innerhalb der Detektorelementmatrix beispielsweise wie folgt berechnet werden: $S_{ij} = W_{ij} \cdot P_{ij}$.

Die Erfinder haben erkannt, dass die Effekte durch unterschiedliche Abschattung des Detektorelements bei unterschiedlichen Fokuspositionen durch Erweiterung bzw. Anpassung der gewichteten Addition reduziert werden können. Die Verteilung der Gewichte kann gezielt angepasst werden, so dass die Effekte durch unterschiedliche Abschattung reduziert werden. Das Ausgleichen kann zu einer derartigen Korrektur der Detektorelementmesswerte führen, so dass die Effekte im nachfolgend erstellten Bild nicht mehr erkennbar oder reduziert sind.

Vorteilhaft können über die statischen Effekte hinaus auch die sich insbesondere zeitlich veränderliche Effekte mittels einer Erweiterung der gewichteten Addition zum Korrigieren des Detektorelementmesswerts ausgeglichen werden. Vorteilhaft können dynamische Einflüsse etwa bedingt durch den Wechsel von der ersten Fokusposition zur zweiten Fokusposition oder rotationsbedingte Verschiebungen bzw. Veränderungen.

Vorteilhaft kann die Bildqualität verbessert werden. Vorteilhaft kann eine Nutzung des Springfokus oder unterschiedlicher Rotationszeiten ermöglicht werden, insbesondere ohne jedes Untersuchungsprogramm bei verschiedenen Rotationszeiten kalibrieren bzw. anpassen zu müssen.

Gemäß einem Aspekt der Erfindung umfasst die Schwankung eine Veränderung einer Abschattung des Detektorelements durch ein Streustrahlenelement.

Bedingt durch die Veränderung von der ersten Fokusposition zur zweiten Fokusposition kann eine Veränderung der Abschattung des Detektorelements durch ein Streustrahlenelement verursacht werden. Der Schattenwurf des Streustrahlenelements kann bei der zweiten Fokusposition gegenüber der ersten Fokusposition verändert sein. Dadurch können, unabhängig vom Untersuchungsobjekt, mehr oder weniger Ereignisse im Detektorelement registriert werden. Vorteilhaft kann durch das erfindungsgemäße Verfahren ein Ausgleich der veränderten Abschattung erreicht werden.

Gemäß einem Aspekt der Erfindung werden die ersten Detektorelementmesswerte oder/und die zweiten Detektorelementmesswerte mittels einer gewichteten Addition ausgeglichen. Die gewichtete Addition kann beispielsweise um einen weiteren Term erweitert werden, welcher die dynamischen Veränderungen bzw. Schwankungen berücksichtigt. Beispielsweise kann eine Summe aus mehreren unterschiedlich gewichteten Summanden jeweils bezogen auf eine Korrektur von statischen Effekten oder dynamischen Schwankungen gebildet werden. Vorteilhaft kann die Schwankung mittels eines zweiten Wichtungsfaktors berücksichtigt und ausgeglichen werden. Vorteilhaft genügt ein vorbestimmter zweiter Wichtungsfaktoren, um die Schwankungen im Wesentlichen auszugleichen. Der erste Wichtungsfaktor und der zweite Wichtungsfaktor können von einem Abstand zwischen der ersten Fokusposition und der zweiten Fokusposition bzw. im Allgemeinen von der Lagedifferenz der ersten Fokusposition und der zweiten Fokusposition abhängen. Beispielsweise kann bei einem Wechsel der Fokusposition mittels eines Springfokus, der Wichtungsfaktor von der Sprungdistanz abhängen.

Gemäß einem Aspekt der Erfindung umfasst die gewichtete Addition eine Antikorrelation innerhalb der Anordnung einer Mehrzahl von Detektorelementen, welche durch ein Streustrahlenelement begrenzt ist, für die erste Fokusposition oder die zweite Fokusposition. Einer Mehrzahl von Detektorelementen kann eine Durchlassöffnung begrenzt durch Streustrahlenelemente zugeordnet sein. Innerhalb der Durchlassöffnung kann eine Anordnung der Mehrzahl von Detektorelementen ausgebildet sein. Die Anordnung kann beispielsweise matrixartig, also in Zeilen und Spalten, ausgebildet sein. Die Anordnung umfasst das Detektorelement. Das erfindungsgemäße Verfahren kann mit einer Mehrzahl von Fokuspositionen durchgeführt werden.

Durch den Wechsel von der ersten Fokusposition zur zweiten Fokusposition kann beispielsweise für ein erstes Detektorelement die Abschattung vergrößert werden und im Wesentlichen gleichen Maß die Abschattung für ein zweites Detektorelement verkleinert werden. Damit können in einem ersten Detektorelement mehr Ereignisse gezählt werden und im zweiten Detektorelement verhältnismäßig weniger Ereignisse gezählt werden. Damit kann beispielsweise die Zählrate von einer Seite des Streustrahlenelements auf die andere Seite des Streustrahlenelements verschoben werden. Die Zählrate kann innerhalb einer Durchlassöffnung entsprechend der Fokusposition auf die Mehrzahl von Detektorelementen verteilt bzw. verschoben werden. Dieses Verhalten kann durch einen Antikorrelationsterm mit dem zweiten Wichtungsfaktor $AC_{ij}$, einen Antikorrelationsfaktor, beschrieben werden, so dass die gewichtete Addition wie folgt erweitert werden kann: $S_{ij} = W_{ij} \cdot P_{ij} + AC_{ij} \cdot P_{ij}$.

Jeder Fokusposition kann ein spezifischer Antikorrelationsterm zugeordnet sein. Es kann für die erste Fokusposition und für die zweite Fokusposition jeweils ein zweiter Wichtungsfaktor $AC_{ij}$ vorbestimmt werden. Bevorzugt sind die zweiten Wichtungsfaktoren $AC_{ij}$ unterschiedlich für die erste Fokusposition und die zweite Fokusposition. Die Werte der zweiten Wichtungsfaktoren unterscheiden sich insbesondere bei Detektorelementen, welche relativ großen Schwankungen unterliegen, für die erste und die zweite Fokusposition. Bei Detektorelementen, beispielsweise mittig innerhalb der Anordnung angeordnet, können die Wichtungsfaktoren für die erste und die zweite Fokusposition ähnlich oder im Wesentlichen gleich sein. Vorteilhaft können die dynamischen Abschattungseffekte beim Wechsel zwischen der ersten Fokusposition und der zweiten Fokusposition ausgeglichen werden.

Gemäß einem Aspekt der Erfindung wird zusätzlich eine zeitlich veränderliche Schwankung ausgeglichen. Die zeitlich veränderliche Schwankung kann entsprechend einer Funktion des Umlaufes oder / und der Rotationsgeschwindigkeit ausgebildet sein. Es können zeitliche Schwankungen der Abschattung durch Verbiegen des Rotors bzw. der Gantry auftreten. Diese Schwankungen können mittels eines zeitlich abhängigen Korrekturterms bzw. Summanden ausgeglichen werden, beispielsweise:

$$S_{ij} = W_{ij} \cdot P_{ij} + AC_{ij}(t) \cdot P_{ij}(t).$$

Zusätzlich kann der erste Wichtungsfaktor bzw. der erste Summand ebenfalls zeitabhängig gewählt werden:

$$S_{ij} = W_{ij}(t) \cdot P_{ij}(t) + AC_{ij}(t) \cdot P_{ij}(t).$$

Damit können beispielsweise zeitliche Schwankungen von Drift- oder Chargesharing-Effekten ausgeglichen werden. Vorteilhaft können zeitliche Effekte, insbesondere vorbestimmte zeitliche Effekte, ausgeglichen werden.

Gemäß einem Aspekt der Erfindung wird die zeitliche Schwankung durch eine Verformung des Röntgendetektors hervorgerufen. Die Verformung des Röntgendetektors kann als Verformung des Rotors, der Detektorvorrichtung oder des Streustrahlengitters ausgebildet sein. Unter Einfluss der Gewichtskraft kann sich während der Rotation der Abstand zwischen der Röntgenquelle und dem Röntgendetektor bzw. dem Streustrahlengitter und dem Röntgendetektor zeitabhängig verändern. Dadurch kann die Abschattung des Detektorelements verändert werden und einer zeitlichen Schwankung entsprechend der Rotationsbewegung unterliegen. Vorteilhaft können zeitliche, mechanische Schwankungen des medizinischen Geräts, insbesondere eines Computertomographiesystems, ausgeglichen werden.

Gemäß einem Aspekt der Erfindung wechseln die erste Fokusposition und die zweite Fokusposition zyklisch. Es kann zwischen einer Mehrzahl von Fokuspositionen, beispielsweise 2, 3 oder 4, zyklisch gewechselt werden. Vorteilhaft können die Schwankungen wiederholt bzw. reproduzierbar auftreten und durch vorbestimmte erste und zweite Wichtungsfaktoren korrigiert werden.

Gemäß einem Aspekt der Erfindung sind die erste Fokusposition und die zweite Fokusposition jeweils vorbestimmt. Die erste Fokusposition und die zweite Fokusposition können in jedem Zyklus im Wesentlichen gleich sein. Damit können auch die Antikorrelationsterme im Wesentlichen für jede Fokusposition immer gleich sein, gegebenenfalls abgesehen von zusätzlichen zeitlichen Schwankungen. Vorteilhaft können die ersten Wichtungsfaktoren und die zweiten Wichtungsfaktoren für alle Szenarien bestimmt werden, beispielsweise vor Auslieferung oder bei einer Systemkalibrierung, und diese dadurch vorbestimmten Wichtungsfaktoren im Betrieb immer wieder verwendet werden.

Gemäß einem Aspekt der Erfindung sind der erste Detektorelementmesswert und der zweite Detektorelementmesswert dem gleichen Energiebereich zugeordnet. Der Röntgendetektor kann mehrere Energiebereiche aufweisen. Dabei kann pro Detektorelement mindestens ein Schwellwert, bevorzugt mehrere Schwellwerte, genutzt werden, um mindestens einen Energiebereich auszubilden. Der Röntgendetektor kann damit energieauflösend Ereignisse registrieren. Der erste Detektorelementmesswert und der zweite Detektorelementmesswert sind dem gleichen Energiebereich bzw. dem gleichen Schwellwert zugeordnet. Alternativ kann der Energiebereich durch ein Röntgenspektrum, beispielsweise beim sogenannten kV-Switching, festgelegt sein. Der erste Detektorelementmesswert und der zweite Detektorelementmesswert sind dem gleichen Röntgenspektrum zugeordnet.

Die Erfindung betrifft ferner einen Röntgendetektor mit einer Mehrzahl von Detektorelementen und ein um die Mehrzahl von Detektorelementen angeordnetes Streustrahlenelement, ferner aufweisend eine Korrektureinheit zum Durchführen eines erfindungsgemäßen Verfahrens. Der Röntgendetektor kann indirektkonvertierend oder insbesondere direkt-konvertierend ausgebildet sein. Der Röntgendetektor kann eine Auswerteeinheit umfassen, welche eine Korrektureinheit zum Ausführen des Verfahrens aufweist. Vorteilhaft kann das Korrekturverfahren direkt im Röntgendetektor durchgeführt werden. Vorteilhaft kann das Korrekturverfahren vor einem möglichen Zusammenfassen von Detektorelementmesswerten mehrerer Detektorelemente durchgeführt werden und damit korrigierte Daten komprimiert innerhalb vom Rotor zum Stator übertragen werden. Der Röntgendetektor kann eine Aufnahmeeinheit, beispielsweise in Form eines Konverterelements, eine Auswerteeinheit und eine Korrektureinheit umfassen.

Die Erfindung betrifft ferner ein medizinisches Gerät aufweisend einen erfindungsgemäßen Röntgendetektor. Gemäß einem Aspekt der Erfindung ist das medizinische Gerät ein Computertomographiesystem. Vorteilhaft können die durch die Bauweise des Computertomographiesystems bedingten Schwankungen reduziert werden.

Die Erfindung betrifft ferner eine Verwendung eines erfindungsgemäßen Röntgendetektors zum Durchführen eines erfindungsgemäßen Verfahrens. Beispielsweise kann ein Röntgendetektor um eine Korrektureinheit erweitert werden bzw. mit einer Korrektureinheit verbunden werden, so dass der Röntgendetektor für das erfindungsgemäße Verfahren verwendet werden kann.

Die Erfindung betrifft ferner ein Computerprogrammprodukt mit einem Computerprogramm, welches direkt in eine Speichereinrichtung einer Steuereinrichtung eines medizinischen Geräts ladbar ist, mit Programmabschnitten, um alle Schritte des erfindungsgemäßen Verfahrens auszuführen, wenn das Computerprogramm in der Steuereinrichtung des medizinischen Geräts ausgeführt wird. Die Erfindung betrifft ferner ein computerlesbares Medium, auf welchem von einer Recheneinheit einlesbare und ausführbare Programmabschnitte gespeichert sind, um alle Schritte des erfindungsgemäßen Verfahrens auszuführen, wenn die Programmabschnitte von der Recheneinheit ausgeführt werden.

Nachfolgend werden Ausführungsbeispiele der Erfindung anhand von Zeichnungen näher erläutert. Hierbei zeigt:

FIG 1 schematisch eine Darstellung des erfindungsgemäßen Verfahrens; und

FIG 2 schematisch eine Darstellung des erfindungsgemäßen medizinischen Geräts.

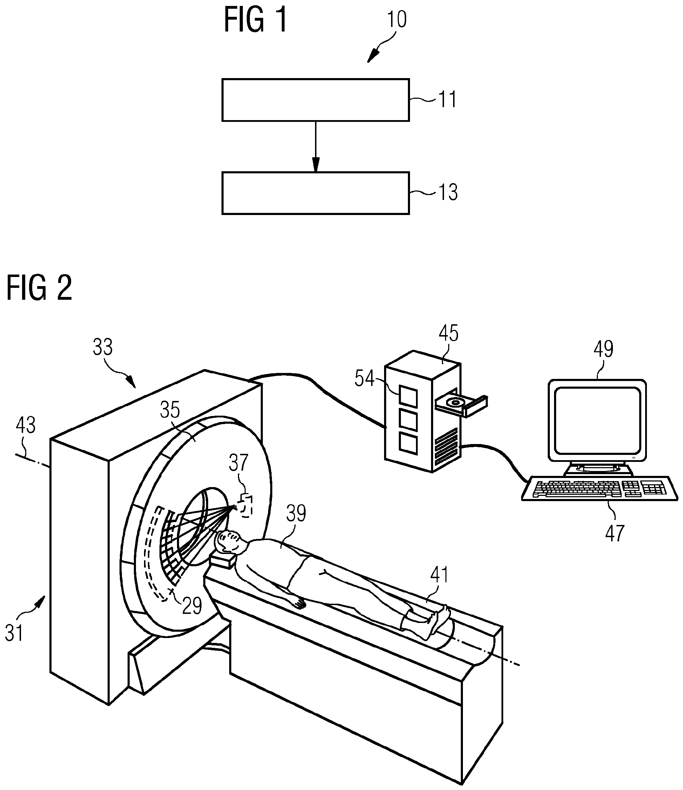

Die Fig. 1 zeigt eine beispielhafte Ausführungsform des erfindungsgemäßen Verfahrens 10. Das Verfahren 10 zur Korrektur von einem ersten Detektorelementmesswert und von einem zweiten Detektorelementmesswert eines Detektorelements eines Röntgendetektors weist die Schritte der Aufnahme 11 und der Korrektur 13 auf. Im Schritt der Aufnahme 11 werden der erste Detektorelementmesswert bei einer ersten Fokusposition einer Röntgenquelle und der zweite Detektorelementmesswert bei einer von der ersten Fokusposition verschiedenen zweiten Fokusposition der Röntgenquelle aufgenommen. Der erste Detektorelementmesswert und der zweite Detektorelementmesswert sind dem gleichen Energiebereich zugeordnet. Im Schritt der Korrektur 13 werden der erste Detektorelementmesswert und der zweite Detektorelementmesswert korrigiert, wobei eine durch den Wechsel von der ersten Fokusposition zur zweiten Fokusposition bedingte Schwankung zwischen dem ersten Detektorelementmesswert und dem zweiten Detektorelementmesswert im Wesentlichen ausgeglichen wird. Die Schwankung umfasst eine Veränderung einer Abschattung des Detektorelements durch ein Streustrahlenelement. Die Information über das Untersuchungsobjekt bleibt dabei im Wesentlichen erhalten.

Der erste Detektorelementmesswert oder/und der zweite Detektorelementmesswert werden mittels einer gewichteten Addition ausgeglichen. Die gewichtete Addition umfasst eine Antikorrelation innerhalb Anordnung einer Mehrzahl von Detektorelementen, welche durch ein Streustrahlenelement begrenzt ist, für die erste Fokusposition oder die zweite Fokusposition. Durch den Wechsel von der ersten Fokusposition zur zweiten Fokusposition kann beispielsweise für ein erstes Detektorelement die Abschattung vergrößert werden und im Wesentlichen gleichen Maß die Abschattung für ein zweites Detektorelement verkleinert werden. Damit können in einem ersten Detektorelement mehr Ereignisse gezählt werden und im zweiten Detektorelement verhältnismäßig weniger Ereignisse gezählt werden. Damit kann beispielsweise die Zählrate von einer Seite des Streustrahlenelements auf die andere Seite des Streustrahlenelements verschoben werden. Die Zählrate kann innerhalb einer Durchlassöffnung entsprechend der Fokusposition auf die Mehrzahl von Detektorelementen verteilt bzw. verschoben werden. Die erste Fokusposition und die zweite Fokusposition sind jeweils vorbestimmt. Dieses Verhalten kann durch einen Antikorrelationsterm mit dem zweiten Wichtungsfaktor $AC_{ij}$, einen Antikorrelationsfaktor, beschrieben werden, so dass die gewichtete Addition wie folgt erweitert werden kann: $S_{ij} = W_{ij} \cdot P_{ij} + AC_{ij} \cdot P_{ij}$.

Zusätzlich wird eine zeitlich veränderliche Schwankung ausgeglichen. Die zeitliche Schwankung wird beispielsweise durch eine Verformung des Röntgendetektors hervorgerufen. Die erste Fokusposition und die zweite Fokusposition wechseln zyklisch. Die zeitlich veränderliche Schwankung kann entsprechend einer Funktion des Umlaufes oder / und der Rotationsgeschwindigkeit ausgebildet sein. Es können zeitliche Schwankungen der Abschattung durch Verbiegen des Rotors bzw. der Gantry auftreten. Diese Schwankungen können mittels eines zeitlich abhängigen Korrekturterms bzw. Summanden ausgeglichen werden, beispielsweise:

$$S_{ij} = W_{ij} \cdot P_{ij} + AC_{ij}(t) \cdot P_{ij}(t).$$

Zusätzlich kann der erste Wichtungsfaktor bzw. der erste Summand ebenfalls zeitabhängig gewählt werden:

$$S_{ij} = W_{ij}(t) \cdot P_{ij}(t) + AC_{ij}(t) \cdot P_{ij}(t).$$

Die Fig. 2 zeigt eine beispielhafte Ausführungsform des erfindungsgemäßen medizinischen Geräts zum Ausführen des erfindungsgemäßen Verfahrens. Das medizinische Gerät ist ein Computertomographiesystem 31. Das Computertomographiesystem 31 beinhaltet eine Gantry 33 mit einem Rotor 35. Der Rotor 35 umfasst eine Röntgenquelle 37 und die Detektorvorrichtung 29. Die Detektorvorrichtung 29 umfasst den Röntgendetektor. Der Röntgendetektor mit einer Mehrzahl von Detektorelementen und ein um die Mehrzahl von Detektorelementen angeordnetes Streustrahlenelement. Das Untersuchungsobjekt 39 ist auf der Patientenliege 41 gelagert und ist entlang der Rotationsachse z 43 durch die Gantry 33 bewegbar. Das Computertomographiesystem 31 umfasst ferner eine Recheneinheit 45. Die Korrektureinheit 54 ist von der Recheneinheit 45 umfasst. Ein computerlesbarer Datenträger mit Programmcode eines Computerprogramms kann von der Recheneinheit 45 eingelesen werden bzw. von der Recheneinheit 45 umfasst sein, um das Verfahren zur Bildgebung, wenn das Computerprogramm auf einem Computer bzw. der Recheneinheit 45 ausgeführt wird, durchzuführen.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann hieraus abgeleitet werden, ohne den Schutzumfang der Erfindung zu verlassen.

**Patentansprüche**

1.  Verfahren (10) zur Korrektur von einem ersten Detektorelementmesswert oder / und von einem zweiten Detektorelementmesswert eines Detektorelements eines Röntgendetektors aufweisend die Schritte

    a) Aufnahme (11) des ersten Detektorelementmesswerts bei einer ersten Fokusposition und des zweiten Detektorelementmesswerts bei einer von der ersten Fokusposition verschiedenen zweiten Fokusposition, und
    b) Korrektur (13) des ersten Detektorelementmesswerts oder/ und des zweiten Detektorelementmesswerts, wobei eine durch den Wechsel von der ersten Fokusposition zur zweiten Fokusposition bedingte Schwankung zwischen dem ersten Detektorelementmesswert und dem zweiten Detektorelementmesswert im Wesentlichen ausgeglichen wird.

2.  Verfahren nach Anspruch 1, wobei die Schwankung eine Veränderung einer Abschattung des Detektorelements durch ein Streustrahlenelement umfasst.

3.  Verfahren nach einem der vorangehenden Ansprüche, wobei der erste Detektorelementmesswert oder/und der zweite Detektorelementmesswert mittels einer gewichteten Addition ausgeglichen werden.

4.  Verfahren nach Anspruch 3, wobei die gewichtete Addition eine Antikorrelation innerhalb Anordnung einer Mehrzahl von Detektorelementen, welche durch ein Streustrahlenelement begrenzt ist, für die erste Fokusposition oder die zweite Fokusposition umfasst.

5.  Verfahren nach einem der vorangehenden Ansprüche, wobei zusätzlich eine zeitlich veränderliche Schwankung ausgeglichen wird.

6.  Verfahren nach Anspruch 5, wobei die zeitliche Schwankung durch eine Verformung des Röntgendetektors hervorgerufen wird.

7.  Verfahren nach einem der vorangehenden Ansprüche, wobei die erste Fokusposition und die zweite Fokusposition zyklisch wechseln.

8.  Verfahren nach einem der vorangehenden Ansprüche, wobei die erste Fokusposition und die zweite Fokusposition jeweils vorbestimmt sind.

9.  Verfahren nach einem der vorangehenden Ansprüche, wobei der erste Detektorelementmesswert und der zweite Detektorelementmesswert dem gleichen Energiebereich zugeordnet sind.

10. Röntgendetektor mit einer Mehrzahl von Detektorelementen und ein um die Mehrzahl von Detektorelementen angeordnetes Streustrahlenelement, ferner aufweisend eine Korrektureinheit zum Durchführen eines Verfahrens nach einem der vorangehenden Ansprüche.

11. Medizinisches Gerät aufweisend einen Röntgendetektor nach Anspruch 10.

12. Medizinisches Gerät nach Anspruch 11, wobei das medizinische Gerät ein Computertomographiesystem (31) ist.

13. Verwendung eines Röntgendetektors nach Anspruch 10 zum Durchführen eines Verfahrens nach einem der Ansprüche 1 bis 9.

14. Computerprogrammprodukt mit einem Computerprogramm, welches direkt in eine Speichereinrichtung einer Steuereinrichtung eines medizinischen Geräts ladbar ist, mit Programmabschnitten, um alle Schritte eines Verfahrens nach einem der Ansprüche 1 bis 9 auszuführen, wenn das Computerprogramm in der Steuereinrichtung des medizinischen Geräts ausgeführt wird.

15. Computerlesbares Medium, auf welchem von einer Rechnereinheit einlesbare und ausführbare Programmabschnitte gespeichert sind, um alle Schritte eines Verfahrens nach einem der Ansprüche 1 bis 9 auszuführen, wenn die Programmabschnitte von der Recheneinheit (45) ausgeführt werden.

FIG 1

FIG 2

Europäisches
Patentamt

European
Patent Office

Office européen
des brevets

# EUROPÄISCHER RECHERCHENBERICHT

**Nummer der Anmeldung**

EP 18 19 7600

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | DE 10 2014 215548 A1 (SIEMENS AG [DE]) 11. Februar 2016 (2016-02-11)<br>* Absatz [0001] *<br>* Absatz [0049] *<br>* Absatz [0055] *<br>* Absatz [0057] - Absatz [0058] *<br>* Absatz [0060] *<br>* Abbildung 6 *<br>----- | 1-15 | INV.<br>G01T1/29<br>A61B6/03 |
| X | JP 2013 009874 A (GE MED SYS GLOBAL TECH CO LLC) 17. Januar 2013 (2013-01-17)<br>* Abbildung 3 *<br>* Absatz [0037] - Absatz [0045] *<br>----- | 1,2 | |
| X | DE 10 2012 214387 A1 (SIEMENS AG [DE]) 27. Februar 2014 (2014-02-27)<br>* Absatz [0039] - Absatz [0041] *<br>* Abbildung 1 *<br>----- | 1,2 | |
| X | US 2011/200169 A1 (OIKAWA SHIRO [JP]) 18. August 2011 (2011-08-18)<br>* Absatz [0063] *<br>* Absatz [0070] - Absatz [0071] *<br>* Abbildung 5 *<br>----- | 1,2 | **RECHERCHIERTE SACHGEBIETE (IPC)**<br><br>G01T<br>A61B |

Der vorliegende Recherchenbericht wurde für alle Patentansprüche erstellt

| Recherchenort | Abschlußdatum der Recherche | Prüfer |
|---|---|---|
| Den Haag | 7. März 2019 | Ordavo, Ivan |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
  ................................................................
& : Mitglied der gleichen Patentfamilie, übereinstimmendes
    Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 18 19 7600

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-03-2019

| Im Recherchenbericht angeführtes Patentdokument | Datum der Veröffentlichung | Mitglied(er) der Patentfamilie | Datum der Veröffentlichung |
|---|---|---|---|
| DE 102014215548 A1 | 11-02-2016 | KEINE | |
| JP 2013009874 A | 17-01-2013 | JP 5863292 B2<br>JP 2013009874 A | 16-02-2016<br>17-01-2013 |
| DE 102012214387 A1 | 27-02-2014 | CN 103584873 A<br>DE 102012214387 A1<br>US 2014042333 A1 | 19-02-2014<br>27-02-2014<br>13-02-2014 |
| US 2011200169 A1 | 18-08-2011 | CN 102160796 A<br>JP 5375655 B2<br>JP 2011167334 A<br>US 2011200169 A1 | 24-08-2011<br>25-12-2013<br>01-09-2011<br>18-08-2011 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102014215548 A1
- DE 102014225399 A1
- DE 102014222855 A1